# EUROPÄISCHE PATENTSCHRIFT

(11) **EP 1 684 775 B1**
(45) Veröffentlichungstag und Bekanntmachung des Hinweises auf die Patenterteilung: **26.03.2008**
(21) Anmeldenummer: 04790852.0
(22) Anmeldetag: 26.10.2004
(51) Int. Cl.: A61K 36/00, A61P 25/24

(54) **VERWENDUNG VON EXTRAKTEN AUS PELARGONIUM-SPEZIES**
USE OF EXTRACTS FROM THE PELARGONIUM SPECIES
UTILISATION D'EXTRAITS DE L'ESPECE PELARGONIUM

(30) Priorität: 29.10.2003 DE 10350338
(43) Veröffentlichungstag der Anmeldung: 02.08.2006
(73) Patentinhaber: Dr. Willmar Schwabe GmbH & Co. KG, 76227 Karlsruhe (DE)
(72) Erfinder: CHATTERJEE, Shyam, Sunder, 76139 Karlsruhe (DE); KOCH, Egon, 76229 Karlsruhe (DE); NÖLDNER, Michael, 76139 Karlsruhe (DE)
(74) Vertreter: Adam, Holger
(86) Internationale Anmeldenummer: PCT/EP2004/012069
(87) Internationale Veröffentlichungsnummer: WO 2005/041993

(56) Entgegenhaltungen:
- WO-A-03/028746
- GB-A- 2 355 189
- GB-A- 2 381 195
- US-A1- 2002 187 115
- HAIDVOGL M ET AL: "AKUTE BRONCHITIS IM KINDESALTER" ZEITSCHRIFT FUER PHYTOTHERAPIE, STUTTGART, DE, Bd. 17, Nr. 5, 1996, Seiten 300,303-304,307, XP008011447
- KOLODZIEJ H ET AL: "PELARGONIUM SIDOIDES DC. NEUESTE ERKENNTNISSE ZUM VERSTAENDNIS DES PHYTOTHERAPEUTIKUMS UMCKALOABO" ZEITSCHRIFT FUER PHYTOTHERAPIE, STUTTGART, DE, Bd. 19, Nr. 3, 1998, Seiten 141-151, XP008011448

## Beschreibung

Die vorliegende Erfindung betrifft die Verwendung von Extrakten aus Pelargonium-Spezies oder deren Pflanzenteilen, insbesondere von P. sidoides und P. reniforme, zur Herstellung eines Arzneimittels zur Prophylaxe oder Behandlung von krankheitsbedingten Verhaltensveränderungen, des chronischen oder postviralen Schwächesyndroms und/oder Stress-induzierter chronischer Krankheitszustände.

Aus persönlicher Erfahrung ist vielen Patienten bekannt, dass Infektionen und Entzündungen, wie z. B. Schnupfen, grippale Infekte oder Infektionen der oberen Atemwege, mit einer Vielzahl von unspezifischen und generalisierten Krankheitssymptomen einhergehen. Neben Erscheinungen wie Fieber und Gelenk- oder Muskelschmerzen gehören dazu auch Verhaltensveränderungen. So kommt es im Zusammenhang mit infektiösen Erkrankungen häufig zu depressiver Verstimmung, Lustlosigkeit, Schwächegefühl, Müdigkeit, Antriebsschwäche, Appetitlosigkeit, sozialer Isolierung, Konzentrationsschwäche, Schlafstörungen, Ängstlichkeit, Interesselosigkeit oder erhöhter Schmerzempfindlichkeit. In ihrer Gesamtheit werden diese Symptome und Verhaltensstörungen als "Akute-Phase-Reaktion", "Sickness Behaviour" oder "Depression aufgrund einer Allgemeinerkrankung" bezeichnet (W. Kozak et al., Am. J. Physiol. 272, R1298 - R1307 (1997); R. Dantzer, Brain Behav. Immun. 15, 7 - 24 (2001); K. W. Kelley et al., Brain Behav. Immun. 17, S112 - S118 (2003); A. H. Miller, Brain Behav. Immun. 17, S132 - S134 (2003)).

Auf molekularer Ebene werden diese Symptome durch die erhöhte Synthese von proinflammatorischen Cytokine, wie z. B. Interleukin-1 (IL-1), IL-6, Tumor-Nekrose-Faktor-α (TNF-α) oder Interferone (INF) hervorgerufen. Diese Mediatoren, die nach Gewebeschädigungen verstärkt produziert werden, lösen indirekt über afferente Nervenbahnen oder direkt nach dem Übertritt ins Gehirn Verhaltensveränderungen aus. Obwohl "Sickness Behaviour" vor allem bei infektionserkrankungen deutlich in Erscheinung tritt, wird es auch im Zusammenhang mit Verletzungen, Traumata, Tumorerkrankungen oder Entzündungsreaktionen, wie z. B. Autoimmunerkrankungen, beobachtet (R. Dantzer, Brain Behav. Immun. 15, 7 - 24 (2001)).

Die Bedeutung von Cytokinen für die Entstehung von unspezifischen Krankheitssymptomen und Verhaltensveränderungen wurde erstmals im Rahmen von klinischen Studien erkannt. Dabei zeigte sich, dass die Verabreichung z. B. von IL-2 oder Interferonen an Patienten mit Tumorerkrankungen, Hepatitis oder Multipler Sklerose Grippe-ähnliche Symptome und psychiatrische Störungen (z. B. akute Psychosen und schwere Depressionen) verursacht. Es gibt inzwischen zahlreiche Hinweise, dass die Cytokin-abhängigen Mechanismen, die zu krankheitsbedingten Verhaltensveränderungen beitragen, auch eine maßgebliche Rolle bei der Pathogenese von Depressionen spielen (L. Capuron und R. Dantzer, Brain Behav. Immun. 17, S119 - S124 (2003)).

In Versuchstieren kann "Sickness Behaviour" durch die direkte Injektion von proinflammatorischen Cytokinen oder die Verabreichung eines Cytokin-Inducers, wie z. B. Lipopolysaccharid, den Zellwandbestandteilen von Gram-negativen Bakterien, hervorgerufen werden. Ähnlich wie bei Menschen sind typische Symptome bei Tieren unter anderem eine reduzierte Futter- und Wasseraufnahme, Gewichtsverlust, verringerte soziale Interaktionen, nachlassendes Sexualverhalten, eingeschränktes Bewegungs- und Explorationsverhalten oder auch fehlendes Interesse an gesüßten Getränken.

Die pathophysiologische Bedeutung von "Sickness Behaviour" liegt vermutlich in einer Anpassung des Organismus an die veränderten Erfordernisse eines erkrankten Organismus. Das hat zur Folge, dass anstrengende körperliche Aktivitäten (z. B. Futtersuche, Sexualverhalten) vermieden und Temperaturverluste eingeschränkt werden (z. B. durch körperliche Ruhe). Gleichzeitig wird die Temperaturproduktion z. B. durch Zittern erhöht. Durch die Summe der Verhaltensveränderungen soll dem Körper der Heilungsprozeß erleichtert werden. Dieser Zustand sollte allerdings nur so lange andauern, wie es der Heilungsprozess erforderlich macht. Tatsächlich sind eine ganze Reihe von Mechanismen bekannt, die die biologischen Effekte der proinflammatorischen Cytokine begrenzen, wie z. B. die erhöhte Synthese von Glucocorticoiden, IL-10 oder α-Melanozyten-stimulierendem Hormon (R. Yirmiya, Current Opinion in Psychiatry 10, 470 - 476 (1997)). Störungen dieser Regulationsmechanismen können zu einem Andauern der immunologischen und neuronalen Prozesse beitragen und zu einer fehlgeleitetenden Anpassungsreaktion führen, die dann als chronisches Schwächesyndrom (Burnout-Syndrome, Chronic Fatiguesyndrom, chronisches Müdigkeitssyndrom) oder postvirales Schwächesyndrom (post-virales Fatigue-Syndrom) zum Ausdruck kommt. Verschiedene Stress-induzierte chronische Krankheitszustände, wie das postraumatische Stress-Syndrom, Fibromyalgie oder multiple Chemikalienempfindlichkeit (Multiple Chemical Sensitivity, Sick Building Syndrome, Electrical Allergy) weisen eine sehr ähnliche Symptomatik auf und viele Patienten erfüllen die diagnostischen Kriterien für eine oder mehrere dieser Erkrankungen. Gemeinsam ist allen, dass sie durch einen Stresszustand ausgelöst werden, der von einem länger andauernden pathologischen Zustand gefolgt wird, wobei der vorausgehende Stress offenbar der Auslöser für einen selbstverstärkenden "Circulus vitiosus" ist. Es wird zunehmend deutlich, dass zwischen dem Nervensystem, dem Immunsystem und dem Hormonsystem enge Verbindungen bestehen und dass alle diese Krankheitszustände durch eine Stressinduzierte verringerte Ansprechbarkeit des Immunsystems auf anti-inflammatorische Signale verursacht werden (M. L. Pall (2001), Med. Hypotheses 57, 139 - 145; G. E. Miller et al. (2002), Health Psychol. 21, 531 - 541).

Wegen ihres häufigen und regelmäßigen Auftretens werden die Symptome des "Sickness Behaviour" von Ärzten häufig ignoriert. Sie werden vielmehr als unangenehme aber nicht zu vermeidende Nebenwirkungen des eigentlichen Krankheitsprozesses angesehen. Durch die in den letzten Jahren gewonnenen Erkenntnisse ist aber klar, daß es sich bei den krankheitsbedingten Verhaltensveränderungen und den damit verbundenen physiologischen Reaktionen (z. B. Fieber) um ein komplexes und eigenständiges pathologisches Geschehen handelt.

Die Symptome von "Sickness Behaviour" können bei betroffenen Patienten einen starken Leidensdruck auslösen und die Lebensqualität entscheidend beeinträchtigen. Insbesondere kann die damit verbundene lethargische Grundhaltung die Mitwirkung des Patienten bei therapeutischen Maßnahmen, z. B. bei Tumorerkrankung, wesentlich erschweren oder den Behandlungserfolg insgesamt in Frage stellen. Bei banalen Erkrankungen, wie z. B. einem grippalen Infekt, steht der Schweregrad der Symptome der krankheitsbedingten Verhaltensveränderungen außerdem häufig in keinem Verhältnis zu dem eigentlichen physiologischen Zweck dieses Abwehrmechanismus.

Durch die Aufklärung der molekularen Mechanismen des "Sickness Behaviours" haben sich in den letzten Jahren neue Angriffspunkte für eine therapeutische Intervention ergeben. Für die Behandlung der depressiven Komponente der krankheitsbedingten Verhaltensveränderungen haben sich Antidepressiva als geeignet erwiesen. Allerdings entfalten Antidepressiva ihre therapeutische Wirkung nur mit einer Verzögerung von mehreren Tagen oder Wochen und induzieren häufig außerdem schwerwiegende Nebenwirkungen. Für akute oder weniger schwerwiegende Erkrankungen sind diese Medikamente daher nicht oder nur eingeschränkt geeignet. Außerdem haben Antidepressiva keinerlei Einfluß auf die neurovegetativen Symptome des "Sickness Behaviours", wie z. B. körperliche Schwäche, Ermüdung oder Appetitlosigkeit. Es besteht deshalb ein dringendes Bedürfnis nach nebenwirkungsarmen und effektiven Behandlungsmethoden für krankheitsbedingte Verhaltensveränderungen.

Extrakte aus den Wurzeln der in Südafrika beheimateten Pelargonium-Spezies P. sidoides und P. reniforme finden in der afrikanischen Volksmedizin eine breite Anwendung zur Behandlung von Diarrhöe, gastrointestinalen Beschwerden, Dysmenorrhoe und Lebererkrankungen. Vorherrschend ist aber die Anwendung bei Atemwegserkrankungen und insbesondere bei Lungentuberkulose. Seit vielen Jahren wird auch ein Extrakt aus den Wurzeln von P. sodoides unter dem Handelsnamen Umckaloabo® kommerziell zur Behandlung akuter und chronischer Infektionen des Hals-, Nasen- und Ohrenbereiches, wie Rhinopharyngitis, Tonsillitis, Sinusitis und Bronchitis vertrieben. Die klinische Wirksamkeit dieses Extraktes scheint auf antimikrobiellen und immunmodulierenden Effekten zu beruhen. Aus experimentellen Untersuchungen gibt es Hinweise, dass Pelargonium sidoides-Extrakt die Synthese von TNF-α, INF-β und Stickoxid (NO) erhöht (H. Kolodziej et al., Phytomedicine 10 (Suppl. 4), 18-24 (2003)).

GB-A-2381195 offenbart die Herstellung von ätherischen Ölen aus Pelargonium gravea und deren Verwendung zur Behandlung von Stress. Aus dieser Druckschrift kann allenfalls die Wirksamkeit von ätherischen Ölen aus Pelargonium gravea gegen Stress, jedoch kein Hinweis auf die Verwendung von Extrakten aus Pelargonium sidoides und Pelargonium reniforme entnommen werden.

Die US-A-2002/0187115 offenbart Zubereitungen aus Pelargonium und deren Verwendung in der Kosmetik und Körperpflege sowie in der allgemeinen Erhaltung der Gesundheit. Hierin werden wiederum essentielle Öle und keine Extrakte ausgewählt aus Pelargonium sidoides und Pelargonium reniforme verwendet.

GB-A-2355189 offenbart eine Zusammensetzung, enthaltend einen oder eine Mixtur aus Extrakten aus verschiedenen Pflanzengattungen, darunter auch von Pelargonium graveolens, sowie die Verwendung solcher Extrakte in der Behandlung verschiedener Krankheitsbilder, welche auch Nervosität, Stress, Ermüdung oder Unterstützung des Immunsystems umfassen. Diese Druckschrift betrifft eine komplexe Kräuterzusammenstellung aus verschiedenen Pflanzen, die nicht auf eine explizite Wirksamkeit von Pelargonium graveolens gegen Nervosität, Stress, Ermüdung oder zur Unterstützung des Immunsystems schließen lassen. Ein Extrakt bestehend aus Pelargonium sidoides und Pelargonium reniforme sowie die erfindungsgemäße Verwendung werden in dieser Druckschrift nicht erwähnt.

WO-A-03/028746 betrifft die Herstellung von Extrakten aus Pelargonium sowie deren Verwendung bei akuten/chronischen Entzündungserkrankungen. Es findet sich jedoch keinerlei Hinweis auf die Behandlung von "Sickness Behaviour".

Haidvogel M. et al., in der Zeitschrift für Phytotherapie, 17(5), 1996, Seiten 300, 303-304, 307-310, 313, beschreiben die erfolgreiche Behandlung der akuten Bronchitis bei Kindern mit dem Phytotherapeutikum "Umckaloabo®". In dieser Arbeit wird aber an keiner Stelle beschrieben, dass es auch ohne eine Verbesserung der respiratorischen Symptome durch Einnahme von Umckaloabo® zu einer positiven Entwicklung der Verhaltensveränderungen kam. Aus dieser Veröffentlichung ergibt sich also kein Hinweis auf eine eigenständig positive Beeinflussung der Symptome von "Sickness Behaviour". Dies gilt gleichermaßen für die Lehre in dem Artikel von Kolodziej H. et al. in der Zeitschrift für Phytotherapie, 19(3), 1998, Seiten 141-151.

Es wurde nun überraschend beobachtet, das Extrakte aus Pelargonien im Tierversuch, trotz der stimulierenden Wirkung auf die Synthese von proinflammatorischen Cytokinen, LPS-induzierte Verhaltensveränderungen positiv beeinflussen und somit zur Prophylaxe und Behandlung von krankheitsbedingten Verhaltensveränderungen ("Sickness Behaviour") bei Mensch und Tier eingesetzt werden können. Bei den krankheitsbedingten Verhaltensveränderungen handelt es sich z. B. um Symptome wie depressive Verstimmung, Lustlosigkeit, Schwächegefühl, Müdigkeit, Antriebsschwäche, Appetitlosigkeit, soziale Isolierung, Konzentrationsschwäche, Schlafstörungen, Ängstlichkeit, Interessenlosigkeit oder erhöhte Schmerzempfindlichkeit, die im zeitlichen Zusammenhang mit Infektionserkrankungen, Verletzungen, Traumata, Tumorerkrankungen, Entzündungsreaktionen oder Autoimmunerkrankungen auftreten. Außerdem eignen sich die Extrakte zur Prophylaxe und Behandlung des "Sickness-Behaviours" im Zusammenhang mit der therapeutischen Anwendung von natürlichen oder rekombinanten Cytokinen, wie z. B. Interleukinen, Interferonen etc. oder auch der Anwendung von Zytostatika oder anderen zell- oder gewebeschädigenden Medikamente und Therapiemaßnahmen. Darüber hinaus können Pelargonium-Extrakte auch zur Vorbeugung und Therapie des chronischen oder postviralen Schwächesyndroms (chronic oder post-viral fatigue syndrom) und verschiedener Stress-induzierter chronischer Krankheitszustände, wie dem postraumatischen Stress-Syndrom, Fibromyalgie oder der multiplen Chemikalienempfindlichkeit verwendet werden.

Extrakte aus Pelargonien oder deren Pflanzenteilen können nach bekannten Herstellungsverfahren in variabler Zusammensetzung mit Lösungsmitteln wie z. B. Wasser, Methanol, Ethanol, Aceton, etc., und deren Gemische bei Temperaturen von Raumtemp. bis 60 °C unter gelinder bis heftiger Durchmischung oder durch Perkolation innerhalb von 10 Min. bis 24 Std. erhalten werden. Bevorzugte Extraktionslösungsmittel sind dabei Gemische von Ethanol und Wasser, besonders bevorzugt im Verhältnis Ethanol / Wasser = 10 / 90 bis 12 / 88 (w/w). Zur Anreicherung wirksamkeitsbestimmender Komponenten können weitere Konzentrierungsschritte durchgeführt werden wie z. B. flüssig-flüssig-Verteilung mit z. B. 1-Butanol/Wasser oder Ethylacetat/Wasser, Adsorption-Desorption an lonenaustauscher, LH20, HP20 und andere Harze oder chromatographische Abtrennungen über RP18, Kieselgel, etc.. Falls die Weiterverarbeitung zu Trockenextrakten erwünscht ist, erfolgt diese nach an sich bekannten Verfahren durch Abziehen des Lösungsmittels bei erhöhter Temperatur und / oder reduziertem Druck oder durch Gefriertrocknung.

Die erfindungsgemäßen Extrakte können in Form von Pulvern, Granulaten, Tabletten, Dragees oder Kapseln oder auch als Lösung, wie sie z. B. bei der Extraktion direkt anfällt, vorzugsweise oral verabreicht werden.

Zur Herstellung von Tabletten wird der Extrakt mit geeigneten pharmazeutisch verträglichen Hilfstoffen wie z. B. Laktose, Cellulose, Siliciumdioxid, Croscarmellose und Magnesiumstearat gemischt und zu Tabletten gepresst, die gegebenenfalls mit einem geeigneten Überzug z. B. aus Hydroxymethylpropylcellulose, Polyethylenglykol, Farbstoffen (z. B. Titandioxid, Eisenoxid) und Talkum versehen werden.

Die erfindungsgemäßen Extrakte können auch, ggf. unter Zusatz von Hilfstoffen wie z. B. Stabilisatoren, Füllmittel etc., in Kapseln abgefüllt werden. Die Dosierung erfolgt dabei so, dass pro Tag 2 bis 1000 mg, bevorzugt 10 bis 200 mg Extrakt zugeführt werden.

Die Wirksamkeit von Pelargonium-Extrakten bei krankheitsbedingten Verhaltensveränderungen und/oder des chronischen oder postviralen Schwächesyndroms wird durch die nachstehend beschriebenen Versuche belegt.

Für die Versuche wurde ein Trockenextrakt aus Wurzeln von P. sidoides verwendet, der mittels zweifacher Mazeration mit der jeweils siebenfachen Menge 11 Gew.-% Ethanol bei 55 °C (11 % Ausbeute) hergestellt wurde. Der Extrakt wurde männlichen NMRI-Mäusen (20 - 25 g Körpergewicht) per Schlundsonde in unterschiedlichen Dosierungen in 0.2 % Agarsuspension (10 ml/kg) verabreicht. Kontrolltiere erhielten nur die Agarsuspension. Eine Stunde nach der Behandlung wurde den Tieren 400 µg/kg Lipopolysaccharid (LPS) (E. coli 0127:B8; Sigma, Deisenhofen) in 10 ml/kg physiologischer Kochsalzlösung (0.9 % NaCl) intraperitoneal injiziert. Nach weiteren zwei Stunden wurden die Tiere in das helle Feld einer Hell-Dunkel-Box gesetzt und die Motilität sowie das Explorationsverhalten über einen Zeitraum von 3 min beobachtet. Als nachtaktive Tiere bevorzugen Mäuse den Aufenthalt in einem dunklen Umfeld. Ein verlängerter Aufenthalt im hellen Bereich der Hell-Dunkel-Box und eine abnehmende Häufigkeit der Wechsel zwischen beiden Feldern ist deshalb als Hinweis auf eine verringertes Explorationsverhalten, Antriebsschwäche und reduziertes Interesse zu werten. Die Ergebnisse der Untersuchungen sind in der nachfolgenden Tabelle zusammengestellt. Es wird deutlich, daß LPS-behandelte Tiere im Vergleich zu Kontrolltieren (NaCl) sich deutlich länger im hellen Bereich aufhalten und weniger häufig zwischen den beiden Kompartimenten wechselen. Diese Wirkung wird durch die Vorbehandlung mit dem Pelargonium-Extrakt dosisabhängig aufgehoben.

**Tab.: Einfluss von Pelargonium-Extrakt auf das Explorationsverhalten von Mäusen in der Hell-Dunkel-Box**

| **Substanz** | **Dosis** mg/kg p.o. | **Aufenthalt im hellen Feld (Sekunden)** MW ± S.D. | **Anzahl der Feldwechsel** |
|---|---|---|---|
| Agar + NaCl | | 83 ± 8 | 8,5 ± 2,8 |
| Agar + LPS | | 135 ± 34 | 4,4 ± 2,8 |
| Pelargonium-Extrakt + LPS | 100 | 119 ± 18 | 5,5 ± 0,8 |
| Pelargonium-Extrakt + LPS | 200 | 104 ± 18* | 5,5 ± 1,4 |
| Pelargonium-Extrakt + LPS | 400 | 97 ± 11 * | 7,5 ± 1,9* |

| | | | |
|---|---|---|---|
| *P<0.05 (= Irrtumswahrscheinlichkeit), t-Test | | | |

## Patentansprüche

1. Verwendung von Extrakten aus Pelargonium-Spezies ausgewählt aus P. sidoides und P. reniforme oder deren Pflanzenteile zur Herstellung eines Arzneimittels zur Prophylaxe oder Behandlung von krankheitsbedingten Verhaltensänderungen, des chronischen oder postviralen Schwächesyndroms und/oder Stress-induzierter chronischer Krankheitszustände.

2. Verwendung nach Anspruch 1, wobei die Pflanzenteile Wurzeln sind.

3. Verwendung nach einem der Ansprüche 1 oder 2, wobei der Extrakt ein wässrig-ethanolischer Extrakt aus Wurzeln von Pelargonium sidoides und/oder reniforme ist.

4. Verwendung nach einem der vorangegangenen Ansprüche 1 bis 3, wobei die krankheitsbedingten Verhaltensveränderungen ausgewählt sind aus den Symptomen depressive Verstimmung, Lustlosigkeit, Schwächegefühl, Müdigkeit, Antriebsschwäche, Appetitlosigkeit, soziale Isolierung, Konzentrationsschwäche, Schlafstörungen, Ängstlichkeit, Interesselosigkeit und erhöhte Schmerzempfindlichkeit, die im zeitlichen Zusammenhang mit Infektionserkrankungen, Verletzungen, Traumata, Tumorerkrankungen, Entzündungsreaktionen oder Autoimmunerkrankungen auftreten.

5. Verwendung nach einem der vorangegangenen Ansprüche 1 bis 4, wobei die krankheitsbedingten Verhaltensveränderungen im Zusammenhang mit der therapeutischen Anwendung von natürlichen oder rekombinanten Cytokinen, wie Interleukinen und Interferonen oder der Anwendung von Zytostatika oder anderen zell- oder gewebeschädigenden Medikamenten und Therapiemaßnahmen stehen.

6. Verwendung nach einem der Ansprüche 1 bis 3, wobei die Stress-induzierten chronischen Krankheitszustände ausgewählt sind aus dem postraumatischen Stress-Syndrom, Fibromyalgie und multipler Chemikalienempfindlichkeit.

## Claims

1. Use of extracts from Pelargonium species selected from P. sidoides and P. reniforme or plant parts thereof for the manufacture of a medicament for the prophylaxis or treatment of disease-related behavioural changes, the chronic or post-viral asthenia syndrome and/or stress-induced chronic pathological conditions.

2. Use according to Claim 1, wherein the plant parts are roots.

3. Use according to any one of claims 1 or 2, wherein the extract is an aqueous-ethanolic extract from roots of Pelargonium sidoides and/or reniforme.

4. Use according to any one of the preceding claims 1 to 3, wherein the disease-related behavioural changes are selected from the symptoms episodes of depression, listlessness, feeling of weakness, fatigue, anergy, anorexia, social isolation, weakness of concentration, sleep disorders, anxiety, indifferentism and hyperalgesia, which occur in a temporal correlation with infectious diseases, injuries, traumata, tumour diseases, inflammatory reactions and autoimmune diseases.

5. Use according to any one of the preceding claims 1 to 4, wherein the disease-related behavioural changes are in connection with the therapeutic application of natural or recombinant cytokines such as interleukins and interferons or with the application of cytostatic agents and other cell-damaging or tissue-damaging medicaments and therapeutic measures.

6. Use according to any one of claims 1 to 3, wherein the stress-induced chronic pathological conditions are selected from posttraumatic stress syndrome, fibromyalgia and multiple chemical sensitivity.

## Revendications

1. Utilisation d'extraits de l'espèce Pelargonium, sélectionnés à partir de *Pelargonium sidoides* et de *Pelargonium reniforme* ou d'autres parties de plante pour la production d'un médicament destiné à la prophylaxie ou au traitement de modifications comportementales dues à une maladie, du syndrome de fatigue chronique ou post-virale et/ou d'états pathologiques chroniques associés au stress.

2. Utilisation selon la revendication 1, les parties de plante étant des racines.

3. Utilisation selon l'une des revendications 1 et 2, l'extrait étant un extrait éthanolique aqueux de racines de Pelargonium sidoides et/ou de Pelargonium reniforme.

4. Utilisation selon l'une des revendications précédentes 1 à 3, les modifications comportementales dues à une maladie étant retenues parmi les symptômes d'humeur dépressive, d'atonie, de sensation de faiblesse, fatigue, débilité motrice, manque d'appétit, isolement social, difficultés de concentration, troubles du sommeil, anxiété, manque d'intérêt et sensibilité exacerbée à la douleur, lesquels surviennent en liaison temporelle avec des pathologies infectieuses, des blessures, traumatismes, affections tumorales, réactions inflammatoires ou maladies auto-immunes.

5. Utilisation selon l'une des revendications précédentes 1 à 4, les modifications comportementales dues à une maladie étant liées à l'utilisation thérapeutique de cytokines naturelles ou recombinantes, telles que des interleukines ou des interférons, ou au recours à des cytostatiques ou à d'autres médicaments et mesures thérapeutiques endommageant les cellules ou les tissus.

6. Utilisation selon l'une des revendications 1 à 3, où les états pathologiques chroniques associés au stress sont retenus parmi le syndrome de stress post-traumatique, la fibromyalgie et la sensibilité chimique multiple.
